# EUROPEAN PATENT APPLICATION

(11) **EP 4 799 555 A1**
(43) Date of publication of application: **02.09.2026**
(21) Application number: 25160204.1
(22) Date of filing: 26.02.2025
(51) Int. Cl.: A61B 5/00

(54) **SYSTEM FOR TREATING A STIFFNESS OR ALLEVIATING A MOVEMENT DISORDER**

(71) Applicant: Point Pressure Italia Srl, 60121 Ancona (IT)
(72) Inventor: ORDOÑEZ REY, Ismael, 60121 Ancona (IT); VERMA, Parikshit, 60121 Ancona (IT); CASELLAS VILADOMS, Marc, 60121 Ancona (IT)
(74) Representative: Hoffmann Eitle

(57) **Abstract**

System configured for treating a stiffness or alleviating a movement disorder caused by a central nervous system injury in a limb of a user. The system comprises a device and a processor. The device comprises attachment means configured for attaching the device to the limb, an action means configured to provide an external input to the limb, an activity sensor configured to measure at least one parameter associated with the user's activity; and a controller configured to control the action means to provide the treatment in the form of external inputs to the limb. The processor is configured to: receive data associated to at least one parameter associated with the user's activity from the activity sensor; and determine the treatment to be provided by the action means through the controller based on the received data associated to the at least one parameter associated with the user's activity.

## Description

### Technical field of the invention

The present invention belongs to the field of medicine. Particularly, the present invention relates to the field of motor disorders associated to the central neural system, more particularly to a system for treating a stiffness or alleviating a movement disorder caused by a central nervous system injury in a limb of a user.

### Background of the invention

Motor disorders resulting from central nervous system (CNS) injuries entail, among many other things, a very high degree of dependence on the patient to perform any motor activity. Some of said common motor disorders are muscle spasticity and muscle stiffness, among others.

Regarding the muscle spasticity, it refers to a muscle control disorder in which the muscles are in a tight or stiffness state and an inability to control those muscles. In addition, reflexes may persist for too long and may be too strong (hyperactive reflexes). For example, an infant with a hyperactive grasp reflex may keep their hand in a tight fist. Spasticity may be caused by an imbalance of signals from the central nervous system (brain and spinal cord) to the muscles. This imbalance is often found in people with cerebral palsy (CP), traumatic brain injury, stroke, multiple sclerosis, spinal cord injury, among others. Treatment for spasticity may include, among others, medications, local injections and occupational and physical therapy programs, involving muscle stretching and range of motion exercises, and sometimes the use of braces, may help prevent tendon shortening. Physical therapy applied to treat spasticity include ultrasound, cryotherapy, vibration, shockwave therapy, magnetic stimulation, transcutaneous electrical nerve stimulation and functional electrical stimulation.

On the other hand, a muscle stiffness condition refers to a sensation of muscle tightness, which often causes pain and makes it a person challenging to move. Muscle stiffness may occur after overuse of a particular muscle, or it may indicate an underlying condition. The muscle stiffness could be treated, depending upon the level of the stiffness, through medicines and/or physical therapy, being the massages, electrotherapy, or acupuncture to most common techniques to reduce or alleviate the stiffness.

However, pharmacological treatments generates tolerance in the individual, preventing them from being a lifelong solution. Physiotherapeutical solutions are resource consuming, requiring qualified professionals and in many cases just tackle the symptoms of the disorder or prevent the worsening of the same.

Lastly, surgery may be used to correct an acute motor disorder. Surgeries such as rhizotomy, neuronal ablation or tendon lengthening are highly invasive and irreversible operations. With neuro-injurious techniques such as "peripheral neurectomy" or with the implantation of a permanent baclofen pump, deformations caused by the motor disorder may be realigned.

However, there is still a need for a personalized, non-invasive, cost-efficient, and long-term solutions for movement disorders caused by central nervous system (CNS) injuries.

### Summary of the invention

A first aspect of the invention relates to a system configured for treating a stiffness or alleviating a movement disorder caused by a central nervous system injury in a limb of a user. The system comprises a device and a processor.

The device comprises i) attachment means configured for attaching the device to the limb, ii) an action means configured to provide an external input to the limb, iii) an activity sensor configured to measure at least one parameter associated with the user's activity; and iv) a controller configured to control the action means to provide the treatment in the form of external inputs to the limb.

The processor is configured to: i) receive data associated to at least one parameter associated with the user's activity from the activity sensor ; and ii) determine the treatment to be provided by the action means through the controller based on the received data associated to the at least one parameter associated with the user's activity.

In a preferred embodiment of the first aspect of the invention, the device further comprises v) at least one force sensor configured to measure the muscle tone of the limb of the user; and the processor is further configured to receive data associated to the muscle tone of the limb of the user from the at least one force sensor and to determine the treatment to be provided by the action means through the controller based on the received data associated to the muscle tone of the limb and the received data associated to the at least one parameter associated with the user's activity.

In a more preferred embodiment of the first aspect of the invention the processor is further configured to determine the need to update the treatment to be provided based on the received data associated to the muscle tone of the limb and to the at least one parameter associated with the user's activity. Further preferably, the processor is further configured to determine the need to update the treatment to be provided during a treatment. In another further preferred embodiment, the processor is further configured to determine the need to update the treatment after a treatment has been performed.

In another preferred embodiment of the first aspect of the invention, the at least one force sensor is a load sensor.

In another preferred embodiment of the first aspect of the invention, the at least one force sensor is a current sensor configured to measure the current employed by the action means.

In another preferred embodiment of the first aspect of the invention, the system comprises at least two force sensors, the two force sensors being a load sensor and a current sensor configured to measure the current employed by the action means.

In another preferred embodiment of the first aspect of the invention, the processor is comprised in an external device.

In another preferred embodiment of the first aspect of the invention, the processor is comprised within the device.

In another preferred embodiment of the first aspect of the invention, the device further comprises communication means to indicate the treatment to be provided and/or the need to update the treatment to be provided.

In another preferred embodiment of the first aspect of the invention, the activity sensor comprises an accelerometer.

In another preferred embodiment of the first aspect of the invention, the action means is configured to provide pressure as an external input to the limb and wherein the processor is configured to control the action means to provide the treatment by pressing the limb at a pre-stablished pressure and at a pre-stablished depth. More preferably, the action means comprises: a) an actuator; and b) a plunger; and the actuator is configured to move the plunger against a user's skin adjacent to the muscle of the limb.

A second aspect of the invention relates to a use of a system according to the first aspect of the invention for treating or alleviating a movement disorder caused by a central nervous system injury in a limb of a user.

A third aspect of the invention relates to a computer program product connected to device according to the first aspect of the invention comprising computer readable instructions which, when executed by a processor, cause the processor to execute the following steps:
a. receiving data associated to the muscle tone of the limb of the user from the force sensor (5) and data associated to at least one parameter associated with the user's activity from the activity sensor (3); and
b. determining the treatment to be provided based on the received data associated to the muscle tone of the limb and to the at least one parameter associated with the user's activity.

A fourth aspect of the invention relates to a method for treating or alleviating a movement disorder caused by a central nervous system injury in a limb of a user. The method comprises:
a. attaching a device according to any one of claims 1 to 14 to the limb of the user,
b. receiving data associated to the muscle tone of the limb of the user from the force sensor (5) and data associated to at least one parameter associated with the user's activity from the activity sensor (3); and
c. determining the treatment to be provided based on the received data associated to the muscle tone of the limb and to the at least one parameter associated with the user's activity.

In a preferred embodiment of the fourth aspect of the invention, the method further comprises:
d. determining the need to update the treatment to be provided based on the received data associated to the muscle tone of the limb and to the at least one parameter associated with the user's activity.

### Brief description of the drawings

To enable a better understanding of the present disclosure, and to show how the present disclosure may be carried out, reference will now be made, by way of example only, to the accompanying schematic drawings, wherein:
Figure 1 shows a schematic view of a system according to one or more embodiments of the first aspect of the invention.
Figure 2 shows a schematic representation of the physiological working mechanism of one or more embodiments of the invention
Figure 3 shows (A) An exploded view and (B) a perspective view of a device according to one or more embodiments of the first aspect of the invention.
Figure 4 shows a diagram of the steps a computer program product is configured to execute according to one or more embodiments of the third aspect of the invention.
Figure 5 shows a diagram of a method for treating or alleviating a movement disorder caused by a central nervous system injury in a limb of a user according to one or more embodiments of the fourth aspect of the invention.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

A first aspect of the invention relates to a system configured for treating a stiffness or alleviating a movement disorder caused by a central nervous system injury in a limb of a user that allows for a personalized, non-invasive, cost-efficient and long-term solution for movement disorders caused by central nervous system (CNS) injuries.

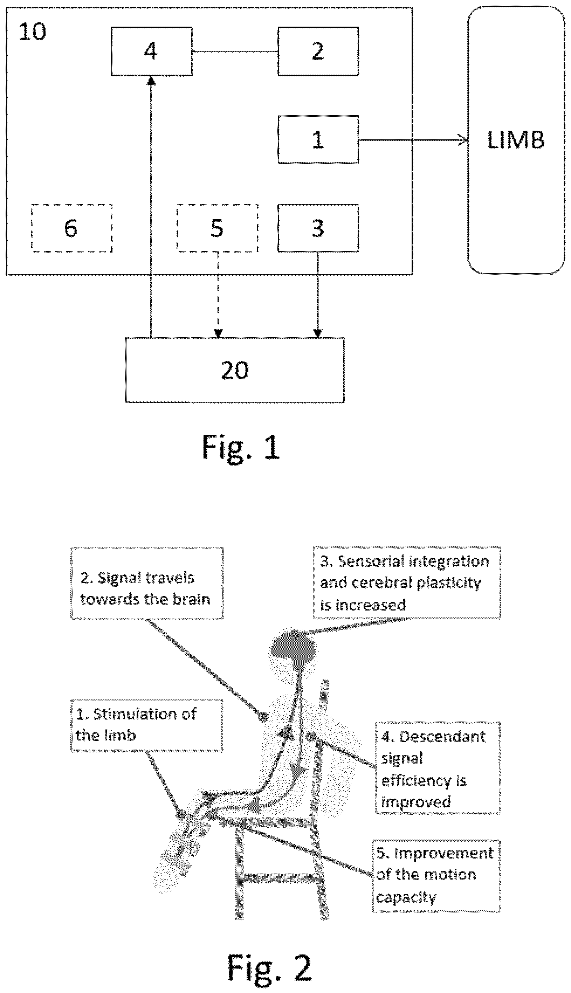

A shown in Fig. 1 the system comprises: a device 10 and a processor 20. The device 10 is configured to be worn or applied to the affected limb, providing targeted action as will be further explained. As will be explained later on, the device 10 incorporates various components comprises attachment means 1, action means 2 an activity sensor 3 and a controller 4.

It is noted that the device 10 may include various other elements, such as a housing or body, and can be designed in different shapes, forms, and sizes to accommodate the specific needs of each user and limb. This versatility allows for customization based on factors like limb size, mobility requirements, and user preferences, making the device adaptable to a wide range of applications. Thus, the device is not limited in particular to any shape, forms and sizes nor to a particular housing or presentation.

The attachment means 1 are configured for attaching the device 10 to the limb of the user. The attachment means may be designed to attach the device to any limb, including but not limited to an arm or a leg. This flexibility allows the system to be applied to various areas of the body affected by stiffness or movement disorders caused by CNS injuries, such as the forearm, upper arm, thigh, or calf, ensuring the device is versatile and adaptable to the specific treatment needs of each user.

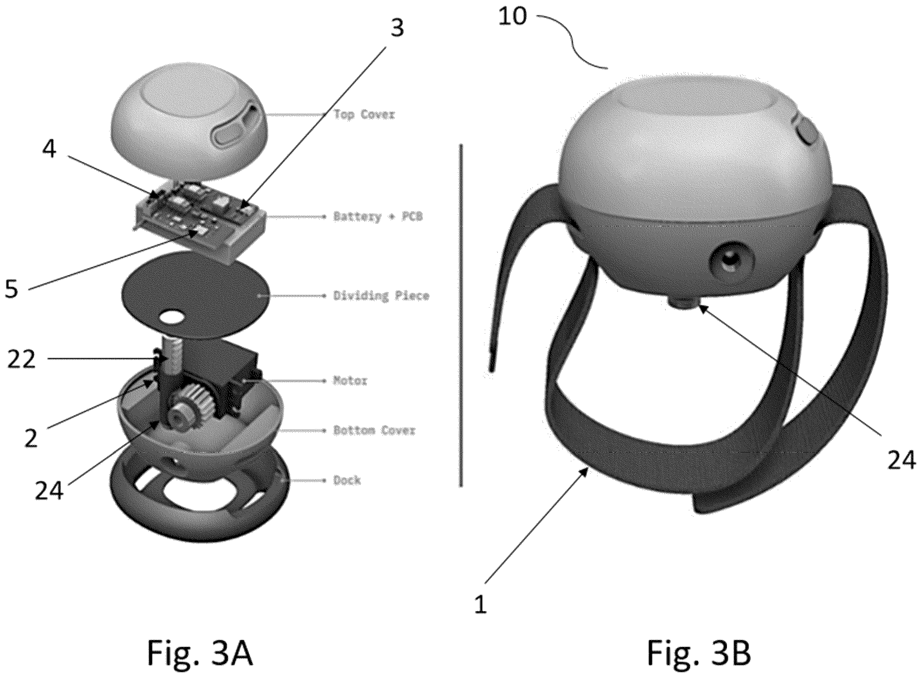

The type of attachment means 1 that may be used include various structures and configurations to securely hold the device in place on the selected limb. As shown in Fig. 3B, in a particular embodiment, the attachment means 1 are straps. Straps are an adaptable attachment type, as they allow for easy adjustment, secure fastening, and comfortable wear, even during extended periods. Many different types of straps may be used, such as Velcro straps, elastic straps, buckle straps, magnetic or snap straps, compression sleeves with integrated straps.

The attachment means 1 may be selected according to the user's preference, limb type, treatment duration, and activity level.

The action means 2 are configured to provide an external input to the limb. The external input to the limb allows for address stiffness or movement disorders resulting from central nervous system (CNS) injuries.

The action means 2 may be any kind of action means that can provide an external input to the limb, such as pressure, temperature, magnetic stimulation, vibration, current or any other alternate the skilled person may envisage.

The action means may be configured in various forms to suit different types of therapy, and each type of action means can be further customized based on the treatment needs. For instance, as noted above, the action means may be configured as a pressure means, as will be explained later on, allowing for therapeutic applications involving rhythmic compression to improve blood flow and reduce stiffness.

The device 10 further comprises an activity sensor 3 configured to measure at least one parameter associated with the user's activity.

The activity sensor 3 may be of different types. Possible types of activity sensors include: accelerometers, gyroscopes, electromyography (EMG) sensors, pressure sensor. The skilled person may envisage many other types of activity sensor 3 all of which are comprised within the present embodiment.

The activity sensor 3 may be configured to measure a variety of parameters associated with the user's activity, as will be explained later. These parameters may include, but are not limited to: movement, range of motion, orientation and/or position of the limb. The skilled person may envisage many other types of parameters that may be measured by the activity sensor 3 all of which are comprised within the present embodiment.

The device 10 further includes a controller 4 configured to control the action means 2 to provide treatment to the limb in the form of external inputs to the limb. The controller 4 plays a central role in managing the therapeutic functions of the system, ensuring that the action means deliver precise, targeted treatment based on the user's specific needs and therapeutic protocol.

The controller 4 may take various forms depending on the system's design and intended functionality. In some embodiments, the controller may be an integrated circuit or dedicated hardware device specifically programmed for control tasks. Alternatively, the controller may be a software-based module within a computing unit or processing system.

The controller 4 is configured to operate the action means 2 by delivering control signals or commands that specify parameters such as intensity, duration, and timing of the treatment. Control can be achieved in various ways, depending on the treatment objectives and user requirements. For example, in one embodiment, the controller may be programmed to deliver treatment according to a predefined plan that is tailored to the user's condition. This plan may involve scheduled activation of the action means at specific intensities and durations. In another embodiment, the controller may adjust the action means based on information received from one or more sensors, such as the activity sensor (3). This real-time responsiveness enables adaptive therapy, promoting safety and effectiveness. Moreover, in some further embodiments, the controller may be configured to receive instructions from an external source, such as a healthcare provider or central processing unit. For example, updated treatment parameters could be sent remotely to modify the treatment plan as the user's condition changes or as new therapeutic data become available. This allows for personalized adjustments without requiring manual intervention by the user.

The system further comprises a processor 20. It is noted that the processor may comprise one or more processing units, such as a microprocessor, GPU, CPU, multi-core processor or the like. Moreover, it is noted that the system may further comprise a memory comprising the instructions to be executed by the processor. The memory may comprise one or more volatile or non-volatile memory devices, such as DRAM, SRAM, flash memory, read-only memory, ferroelectric RAM, hard disk drives, floppy disks, magnetic tapes, optical disks or the like. The controller may be implemented in software (a computer program), hardware (a physical device), or any combination in order to execute the sequences of operation disclosed herein. Moreover, as will be explained later, the processor may be comprised within the device 10, or may be outside of the device 10, such as in an external device or in a remote device.

The processor 20 is configured to:
i. receive data associated to at least one parameter associated with the user's activity from the activity sensor 3.
   It is noted that the processor 20 may receive one or more data points from the activity sensor 3, depending on the number and type of parameters being monitored. This data can include multiple distinct measurements such as movement, muscle activation, heart rate, or other physiological metrics, providing a comprehensive view of the user's current state. Moreover, the data received by the processor 20 may either be raw measurements directly from the sensor or aggregated data. In some cases, the activity sensor 3 may combine multiple measurements to compute a specific parameter. For example, a movement parameter might be derived from both accelerometer and gyroscope data, where the sensor combines and processes these values to provide a single, unified measure of movement quality or intensity. In other cases, the data received by the processor may be computed as a function of multiple parameters captured by different sensors. The skilled person may envisage many different alternatives in which the processor may receive the data associated to at least one parameter associated with the user's activity from the activity sensor 3, all of which are comprised within the first aspect of the invention.
ii. determine the treatment to be provided by the action means 2 through the controller 4 based on the received data associated to the at least one parameter associated with the user's activity.
   The data received from the activity sensor 3 provides insights into the user's movement patterns, physiological responses, and any deviations from a desired state. The processor 20 can interpret this information to determine how the action means 2 should be adjusted. Several methods can be used to compute the appropriate treatment based on the activity data. In some embodiments, the processor 20 may apply an algorithm to analyse the activity data and calculate specific treatment adjustments. This algorithm may consider multiple parameters and compute an optimal response from the action means 2. The algorithm may incorporate real-time adjustments, continually updating the treatment to match changes in the user's activity. In other embodiments, the processor 20 may refer to a predefined table to determine treatment settings based on the activity data. This table could include combinations of input parameters and their corresponding treatment responses. For example, a range of activity levels or sensor readings could be matched to specific therapy intensities or types. In certain cases, the processor may rely on predefined thresholds to guide treatment. For example, if a parameter exceeds a specific threshold, the processor could automatically reduce the intensity or pause the treatment to ensure the user's safety. Thresholds can be established for each parameter independently, allowing for responsive and condition-specific adjustments.
   The algorithms, tables, and thresholds used for treatment determination may be preselected or customized according to individual user profiles. Factors such as the user's physiognomy, underlying medical conditions, specific injury characteristics, and the limb being treated (e.g., arm or leg) can influence these preselected parameters. By taking into account these personalized factors, the processor 20 ensures that the treatment delivered by the action means 2 is not only accurate and effective but also safe and suitable for each individual user.

Advantageously, a system configured for treating a stiffness or alleviating a movement disorder caused by a central nervous system injury in a limb of a user according to the first aspect of the invention, provides for a personalized solution for movement disorders caused by central nervous system (CNS) injuries, since the treatment is tailored at least according to the user's activity. It also provides for a non-invasive solution for movement disorders caused by central nervous system (CNS) injuries, since the system does not invade the user, and does not require an expert to be used. Moreover, is a cost-efficient solution for movement disorders caused by central nervous system (CNS) injuries since it can be reused for each session without fungibles for most of their use, while adapting to the user's needs as determined by the activity parameter. Finally, it is also and long-term solution for movement disorders caused by central nervous system (CNS) injurie, since it does not generate tolerance, and can be adapted to the user's evolution.

As Shown in Fig 2, the entry of maximum information occurs at those points where anatomically more receptors are collected, and where the greatest number of motor plates or nerve terminals (from the brain to the muscle). This is where, through periodic, sustained, and calibrated pressures on the tissue, they stimulate this input of information that our system will need for the movement that we will later need, such as walking. This is the basic principle, motor control and learning through the peripheral input of sensory information. The stimuli travel as a signal to the brain wherein the sensorial integration and cerebral plasticity is increase, achieving an improvement in the efficiency of the afferent signal, which in turn increases the ability of performing the movement that will be late needed.

Figs, 3A and 3B show an exploded view and perspective view of a device 10 according to one or more embodiments of the invention, respectively. It is noted that the device 10 disclosed in Figs. 3A and 3B comprise particular implementations of one or more embodiments of the first aspect of the invention and may comprise additional features that are optional according to the first aspect of the invention. Therefore, Figs. 3A and 3B shall not be considered as limiting the first aspect of the invention, and the skilled person may envisage many different alternative of the same according to the first aspect of the invention.

It is noted that any system according to the first aspect of the invention may comprise more than one device 10. Therefore, the system comprises more than one device 10 and the treatment to be provided by the action means 2 through the controller 4 of each device 10 is controlled by the processor 20. In some embodiments the system may comprise more than one processor 20 too, for example, one processor 20 for each device 10.

According to a preferred embodiment of the first aspect of the invention, and as shown in Fig. 1, the device 10 further comprises at least one force sensor 5 configured to measure the muscle tone of the limb of the user.

Muscle tone is the continuous, passive partial contraction of the muscles or the muscle's resistance to passive stretch during resting state. It reflects the level of muscle tension or readiness, playing a key role in posture and movement control. For users with CNS injuries, muscle tone can be either elevated (hypertonia) or reduced (hypotonia), affecting the user's range of motion and control over their movements.

The force sensor 5 may encompass different types of force sensors, such as load sensors, or current sensors associated to the action means 2 as will be explained later on. In some implementations the device 10 may include more than one force sensor (5) to allow for a comprehensive assessment of muscle tone, either to cross validate data or to assess the muscle tone across different muscle groups or regions of the limb. These force sensors can be of the same type or different types (e.g., a combination of load and current sensors) to provide both direct and indirect measures of muscle tone. This flexibility enables the device to gather diverse and detailed data on muscle function, enhancing the accuracy of muscle tone assessments.

The force sensor 5 measures the muscle tone by detecting the amount of resistance exerted by the muscle against the sensor, providing quantifiable data on muscle tension. For instance, a load sensor can measure the force applied when the muscle is passively stretched or actively contracted, allowing the device to assess muscle tone.

As the attachment means 1 are configured for attaching the device 10 to the user's limb, the device is able to securely position the force sensor 5 against the relevant muscle groups. This secure positioning enables precise measurements of muscle tone in the specific limb area, ensuring accurate data collection for monitoring muscle tone.

It is noted that in some embodiments the force sensor 5 may be understood as a pressure sensor for a given known surface of contact. Therefore, herein after reference will we made to a force sensor 5. but, as the skilled person knows, the force sensor 5 can also be referred to as a pressure sensor.

According to this preferred embodiment, the processor 20 is further configured to receive data associated to the muscle tone of the limb of the user from the at least one force sensor 5. Therefore, the processor 20 in addition to data associated to at least one parameter associated with the user's activity from the activity sensor 3, it is configured to receive data associated to the muscle tone of the limb of the user from the at least one force sensor 5.

The processor may aggregate data from both the activity sensor 3 and the force sensor 5. For example, it might combine muscle tone data (force sensor 5) with movement data (activity sensor 3) to assess how the user's muscle tone is influencing or being influenced by their movement patterns. Aggregation allows the system to track complex, interdependent variables in real-time and makes it possible to evaluate the user's condition holistically. Moreover, the processor may combine data from both sensors in a more analytical way, processing them through a computational model or algorithm.

The skilled person may envisage many different alternatives in which the processor may receive the data associated to at least one parameter associated with the user's activity from the activity sensor 3, all of which are comprised within the first aspect of the invention.

According to this preferred embodiment, the processor 20 is configured to determine the treatment to be provided by the action means 2 through the controller 4 based on the received data associated to the muscle tone of the limb and the received data associated to the at least one parameter associated with the user's activity.

Several methods can be used to compute the appropriate treatment based on the muscle tone data and the activity data. In some embodiments, the processor 20 may apply an algorithm to analyse the muscle tone data and the activity data and calculate specific treatment adjustments. This algorithm may consider multiple parameters and compute an optimal response from the action means 2. The algorithm may incorporate real-time adjustments, continually updating the treatment to match changes in the muscle tone and/or the user's activity. In other embodiments, the processor 20 may refer to a predefined table to determine treatment settings based on the muscle tone data and the activity data. This table could include combinations of input parameters and their corresponding treatment responses. For example, a range of activity levels or sensor readings could be matched to specific therapy intensities or types. In certain cases, the processor may rely on predefined thresholds to guide treatment. For example, if a parameter exceeds a specific threshold, the processor could automatically reduce the intensity or pause the treatment to ensure the user's safety. Thresholds can be established for each parameter independently, allowing for responsive and condition-specific adjustments.

The algorithms, tables, and thresholds used for treatment determination may be preselected or customized according to individual user profiles. Factors such as the user's physiognomy, underlying medical conditions, specific injury characteristics, and the limb being treated (e.g., arm or leg) can influence these preselected parameters. By taking into account these personalized factors, the processor 20 ensures that the treatment delivered by the action means 2 is not only accurate and effective but also safe and suitable for each individual user.

Advantageously, a system configured for treating a stiffness or alleviating a movement disorder caused by a central nervous system injury in a limb of a user according to this preferred embodiment of the first aspect of the invention, further provides for a personalized solution for movement disorders caused by central nervous system (CNS) injuries, since the treatment is tailored at least according to the user's activity and his/her muscle tone. It also provides for a non-invasive solution for movement disorders caused by central nervous system (CNS) injuries, since the system does not invade the user, and does not require an expert to be used. Moreover, is a cost-efficient solution for movement disorders caused by central nervous system (CNS) injuries since it can be reused for each session without fungibles for most of their use, while adapting to the user's needs including the user's evolution as determined by the activity parameter and the muscle's tone. Finally, it is also and long-term solution for movement disorders caused by central nervous system (CNS) injurie, since it does not generate tolerance, and can be adapted to the user's evolution.

According to a more preferred embodiment of the first aspect of the invention, the processor 20 is further configured to determine the need to update the treatment to be provided based on the received data associated to the muscle tone of the limb and/or to the at least one parameter associated with the user's activity.

The data received from both the muscle tone (via the force sensor 5) and the activity parameters (via the activity sensor 2) provides key insights into the user's recovery, condition progression, and current needs. These parameters reflect the user's real-time physiological state and activity level, allowing for dynamic and personalized adjustments in treatment.
if the activity sensor 3 detects that the limb is in a particular position, such as a flexed or extended posture, this could indicate the user's current mobility capabilities or limitations. If the user attempts to hold or move the limb in a position that is challenging, it may suggest that muscle tone is insufficient to support the required action. Alternatively, if muscle tone is high, it could indicate spasticity or stiffness. The processor 10 can adjust treatment based on the current position of the limb and whether the user's muscles are responding adequately to that position. The muscle tone as determined by the force sensor 5 may also provide information on the user's condition. If the muscle tone is elevated (hypertonia) or reduced (hypotonia) with respect to the previous known tone, it may be indicative of a need to update the treatment to be provided

By integrating the data associated with muscle tone and/or the user's activity parameters, the processor 20 is able to continuously monitor the user's current condition. This integrated information provides a comprehensive view of the user's status, allowing for more precise treatment updates. The processor can determine if the current treatment needs adjustment to better address the user's immediate requirements, either by increasing, decreasing, or modifying the type of external input provided by the action means 2 through the controller 4.

The need to update the treatment to be provided based on the received data associated to the muscle tone of the limb and/or to the at least one parameter associated with the user's activity can be computed according to several methods, such as algorithms, predefined look-up tables and or predefined thresholds as already mentioned in previous embodiments.

Moreover, the algorithms, tables, and thresholds used for treatment update determination may be preselected or customized according to individual user profiles as already mentioned in previous embodiments. By taking into account these personalized factors, the processor 20 ensures that the update of the treatment delivered by the action means 2 is not only accurate and effective but also safe and suitable for each individual user.

Advantageously, this further provides for a more personalized solution for movement disorders caused by central nervous system (CNS) injuries, since the treatment is tailored according to the user's activity and/or his/her muscle tone through time.

In a particular preferred embodiment of the first aspect of the invention, when the device 10 further comprises at least one force sensor 5 configured to measure the muscle tone of the limb of the user, the processor 20 is further configured to determine the need to update the treatment to be provided based on the received data associated to the muscle tone of the limb and to the at least one parameter associated with the user's activity.

Advantageously, it further provides for a more personalized solution for movement disorders caused by central nervous system (CNS) injuries, since the treatment is tailored according to the user's activity and/or his/her muscle tone through time.

According to a furthermore preferred embodiment of the first aspect of the invention, the processor 20 is further configured to determine determine the need to update the treatment to be provided during a treatment.

During the course of treatment, the muscle stiffness and/or the user's activity may indicate that the originally planned treatment is not as effective as intended, requiring an update to the treatment strategy. This dynamic adaptability is crucial to ensuring that the treatment remains optimal and personalized to the user's evolving needs.

As the muscle stiffness and the user's activity can change in real time, the processor can use this information to adjust the treatment during the session. If the processor detects that the user is encountering difficulty during an activity it may adjust the treatment to make it more effective, either by increasing or decreasing the intensity of stimulation, switching between different action means, and/or modifying the treatment parameters to target a specific issue.

The need to update the treatment to be provided based on the received data associated to the muscle tone of the limb and/or to the at least one parameter associated with the user's activity can be computed according to several methods, such as algorithms, predefined look-up tables and or predefined thresholds as already mentioned in previous embodiments.

Moreover, the algorithms, tables, and thresholds used for treatment update determination may be preselected or customized according to individual user profiles as already mentioned in previous embodiments. By taking into account these personalized factors, the processor 20 ensures that the update of the treatment delivered by the action means 2 during a treatment is not only accurate and effective but also safe and suitable for each individual user.

Advantageously, a system according to this more furthermore preferred embodiment of the first aspect of the invention, further tailors the treatment during a treatment, which increases the adaptability of the system without requiring any modification of the same, further making it more cost-efficient.

According to another furthermore preferred embodiment of the first aspect of the invention, the processor is further configured to determine the need to update the treatment after a treatment has been performed.

The need to update the treatment to be provided based on the received data associated to the muscle tone of the limb and/or to the at least one parameter associated with the user's activity can be computed according to several methods, such as algorithms, predefined look-up tables and or predefined thresholds as already mentioned in previous embodiments.

Moreover, the algorithms, tables, and thresholds used for treatment update determination may be preselected or customized according to individual user profiles as already mentioned in previous embodiments. By taking into account these personalized factors, the processor 20 ensures that the update of the treatment delivered by the action means 2 after a treatment is not only accurate and effective but also safe and suitable for each individual user.

Advantageously, this allows for a system that is able to personalise the user's treatment without requiring from an expert input, such that of a physician, which makes it more cost-efficient solution without losing personalization.

According to another preferred embodiment of the first aspect of the invention, the at least one force sensor (5) is a load sensor.

According to another preferred embodiment of the first aspect of the invention, the at least one force sensor 5 is a current sensor configured to measure the current employed by the action means 2.

By analyzing the current required to maintain this applied pressure, the system can infer the muscle tone of the user's limb. When muscles are stiffer or have increased tone, they resist external pressure more strongly. This increased resistance means that the action means 2 must use more current to maintain a consistent level of pressure against the muscle.

Conversely, if the muscle tone is lower and the muscles are more relaxed, less current is required by the action means 2 to maintain the same pressure. Thus, the measured current serves as an indirect indicator of the muscle tone: higher current reflects increased muscle tone or stiffness, while lower current indicates reduced muscle tone.

Advantageously, the device 10 is simplified as the muscle tone of the limb of the user can be measured indirectly through the action means 2.

According to another preferred embodiment of the first aspect of the invention, the system comprises at least two force sensors 5, the two force sensors being a load sensor and a current sensor configured to measure the current employed by the action means 2.

In this embodiment, data associated with the muscle tone of the limb can be computed as a function of the measurements received from both the load sensor and the current sensor, allowing for a more precise and multifaceted assessment of muscle tone.

By integrating the data from both sensors, the system can compute a more comprehensive measure of muscle tone. For example, if both the load sensor and the current sensor indicate high readings, this would strongly suggest elevated muscle stiffness. If one sensor detects a high value while the other is low, the system can interpret these combined data points to refine its understanding of the muscle's condition, providing a nuanced view of muscle tone.

Advantageously, the device 10 can measure the muscle tone of the limb of the user with more precision, which in turn ensures the treatment to be provided can be determined with more precision.

According to another preferred embodiment of the first aspect of the invention, the processor 20 is comprised in an external device.

The external device housing the processor 20 can take various forms, such as: a wearable control unit, a handheld device such as a smartphone or a dedicated remote, a computer or even a cloud-based server.

These external device configurations provide varied levels of portability, processing capability, and interactivity, allowing the system to be tailored to different user needs, environments, and clinical requirements.

Advantageously, the device 10 can be more space-efficient and energy efficient, as the processor configured to determine the treatment to be provided by the action means (2) through the controller 4 is external to the device 10. As the device is more spatial and energy efficient, this further increases the comfort of the device 10, as it is less heavy, less bulky, and its battery lasts longer.

According to another preferred embodiment of the first aspect of the invention, the processor 20 is comprised within the device 10.

Advantageously, the system does not depend on external devices as it is self-contained in the device 10.

According to another preferred embodiment of the first aspect of the invention, the device 20 further comprises communication means 6 to indicate the treatment to be provided and/or the need to update the treatment to be provided.

The communication means 6 can encompass various types of communication technologies and protocols according to the need of the system. It may comprise more than one communication means, each serving distinct purposes or enhancing the system's reliability and adaptability. Possible types of communication means include any kind of wireless communication means, such as Bluetooth, Wi-Fi, NFC or Zigbee. The skilled person may envisage many alternatives and protocols the system may use as communication means 6, all of which are comprised within the present invention.

Advantageously, the system can inform the user or a clinician the treatment to be provided determined according to the processor based on the received data associated to the at least one parameter associated with the user's activity

According to another preferred embodiment of the first aspect of the invention, the activity sensor 3 comprises an accelerometer.

An accelerometer can serve as a valuable input for determining the treatment to be provided by capturing real-time data on the movement, orientation, and acceleration of the user's limb. This data can be processed to assess various aspects of the user's activity level and physical state, which, in turn, helps in customizing the treatment. It is noted that the accelerometer, may be comprised within a more complex sensor such as an IMU.

According to another preferred embodiment of the first aspect of the invention, the action means 2 is configured to provide pressure as an external input to the limb and the processor 20 is configured to control the action means 2 to provide the treatment by pressing the limb at a pre-stablished pressure and at a pre-stablished depth.

The pre-stablished pressure and at a pre-stablished depth of the treatment may be predefined according to the user's physiognomy and/or condition or may be determine according to a calibration procedure to further tailor it to the specific need of the user at the moment the system is to be used.

In a more preferred embodiment, the calibration procedure is performed by the controller 4 controlling the action means 2 to cause the plunger to apply a pressure from an initial pressure value to a threshold pressure value, setting the pre-stablished pressure and the pre-stablished depth at the threshold pressure value.

The threshold pressure value can be stablished trough the user pain feedback. As the plunger starts to press the specific point of the muscle, the initial pressure value "no pain" or "lower pain" for the user is set (e.g., as the plunger slightly touches the skin). The plunger continues pressing the muscle, sinking in the skin, until reaching the "umbral pain" or "higher pain" that the user can resist, wherein this "umbral pain" or "higher pain" sets the threshold pressure value. Since the level of pain is unique for every user, once the initial and the threshold pressure value are established and, consequently, the pre-stablished pressure and the pre-stablished depth for the user are stablished, being these data stored in the data storage means associated to a user profile for management and later therapies.

Alternatively, through the force sensor, the pre-stablished pressure and the pre-stablished depth can be set. For example, 0 (Zero) value of pressure (as the plunger slightly touches the skin) corresponds the initial pressure value, and the threshold pressure value can be established when the force sensor are scoring, for example, 6 Kgf. In this case, the threshold pressure value, can be settled by medical recommendations or as "umbral pain" previously stablished of the user, among others.

Advantageously, the device could be used for treating multiple users, applying the proper therapy for each user profile.

According to a more preferred embodiment of the first aspect of the invention, and as shown in Fig. 3A the action means 2 comprises: an actuator 22 and a plunger 24, and the actuator 22 is configured to move the plunger 24 against a user's skin adjacent to the muscle of the limb.

The actuator 22 is a component designed to provide controlled mechanical movement or force. In this embodiment, the actuator drives the plunger 24, applying the necessary action to the user's limb. The actuator 22 may be electric, pneumatic or hydraulic. Actuators can be manufactured in various sizes and shapes, allowing customization to suit different treatment needs and user limb sizes. Actuator shape and size are selected based on the force required, space constraints, and the level of precision necessary for the treatment.

The plunger 24 is a movable component that applies direct pressure or input to the user's limb. The plunger transmits the force generated by the actuator, facilitating therapeutic actions such as pressure application, stretching, or movement assistance. The plunger 24 may be a fixedhead, spring-loaded or adjustable.

Plungers can come in various sizes and shapes to match the specific anatomy and treatment needs of the user. A larger, flat-surfaced plunger may be suitable for broad pressure application over larger muscle areas, while a smaller, round or pointed plunger may target precise pressure points. Shape considerations include flat, rounded, or even concave designs, each suited for specific types of therapeutic engagement with the muscle or tissue.

In Fig. 3a the action means 2 comprising the actuator 22 and the plunger 24 is located in a particular place but it can be placed in different places according to the limb the device 10 is configured to be attached to, or according to the treatment to be provided, for example, according to the muscle to be treated.

A second aspect of the invention relate to the use of a system according to any one of the embodiments of the first aspect of the invention for treating or alleviating a movement disorder caused by a central nervous system injury in a limb of a user.

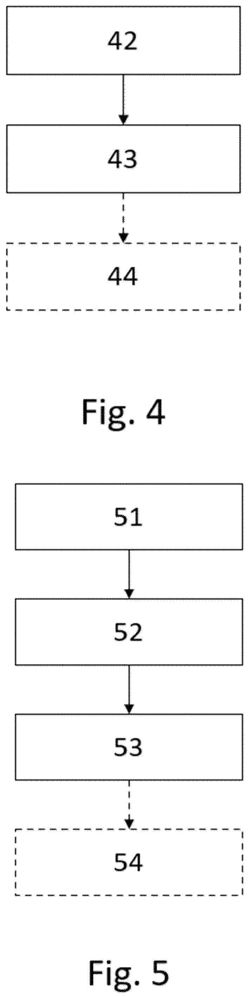

A third aspect of the invention relates to a computer program product connected to device according to any one of the embodiments of the first aspect of the invention, comprising computer readable instructions which, when executed by a processor, preferably the processor 20, cause the processor to execute the following steps as shown in Fig. 4:
In a first step 42, it causes the processor to receive data associated to the muscle tone of the limb of the user from the force sensor 5 and/or data associated to at least one parameter associated with the user's activity from the activity sensor 3.

In a second step 43 it causes the processor to determine the treatment to be provided based on the received data associated to the muscle tone of the limb and/or to the at least one parameter associated with the user's activity.

It is noted that the step 43 may be perform in many ways such as combining data from the force sensor 5 and the activity sensor 3, from a plurality of sensors of any type, and may be done during or after the treatment being provided, as explained with respect to the embodiment of the first aspect of the invention.

The treatment to be provided based on the received data associated to the muscle tone of the limb and/or to the at least one parameter associated with the user's activity can be computed according to several methods, such as algorithms, predefined look-up tables and or predefined thresholds as already mentioned in the embodiments of the first aspect of the invention.

Moreover, the algorithms, tables, and thresholds used for treatment determination may be preselected or customized according to individual user profiles as already mentioned in previous embodiments. By taking into account these personalized factors, the processor ensures that the determination of the treatment to be delivered by the action means 2 is not only accurate and effective but also safe and suitable for each individual user.

Advantageously, the computer program product is able to configure a system according to any one of the embodiments of the first aspect of the invention for treating a stiffness or alleviating a movement disorder caused by a central nervous system injury in a limb of a user.

In a preferred embodiment, when the device 10 further comprises at least one force sensor (5) configured to measure the muscle tone of the limb of the user, the computer program product comprises computer readable instructions which, when executed by a processor, cause the processor to execute the following steps, as shown in Fig. 4:
In a first step 42, it causes the processor to receive data associated to the muscle tone of the limb of the user from the force sensor 5 and data associated to at least one parameter associated with the user's activity from the activity sensor 3.

In a second step 43 it causes the processor to determine the treatment to be provided based on the received data associated to the muscle tone of the limb and to the at least one parameter associated with the user's activity.

In a more preferred embodiment of the third aspect of the invention, and as shown in Fig. 4 the computer program product comprises computer readable instructions which, when executed by a processor, cause the processor to further execute the step 54 of determining the need to update the treatment to be provided based on the received data associated to the muscle tone of the limb and/or the at least one parameter associated with the user's activity.

The update on the treatment to be provided based on the received data associated to the muscle tone of the limb and/or to the at least one parameter associated with the user's activity can be computed according to several methods, such as algorithms, predefined look-up tables and or predefined thresholds as already mentioned in the embodiments of the first aspect of the invention.

A fourth aspect of the invention relates to a method for treating or alleviating a movement disorder caused by a central nervous system injury in a limb of a user. As shown in Fig. 5 the method comprises the following steps:
In a first step 51, the method involves attaching a device according to any one of the embodiments of the first aspect of the invention to the limb of the user. This attachment may be achieved through the attachment means 1 as described in the embodiments of the first aspect of the invention. Specifically, the attachment means may include various structures, such as adjustable straps, bands, or other securing mechanisms, that ensure the device remains in place on the limb during treatment. As above mentioned, the attachment means may be designed to attach the device to any limb, including but not limited to an arm or a leg.

This flexibility allows the system to be applied to various areas of the body affected by stiffness or movement disorders caused by CNS injuries, such as the forearm, upper arm, thigh, or calf, ensuring the device is versatile and adaptable to the specific treatment needs of each user.

In a second step 52, the method involves receiving data associated to the muscle tone of the limb of the user from the force sensor 5 and data associated to at least one parameter associated with the user's activity from the activity sensor 3

Lastly, in a third step 53, the method involves determining the treatment to be provided based on the received data associated to the muscle tone of the limb and to the at least one parameter associated with the user's activity.

It is noted that the step 53 may be perform in many ways such as combining data from the force sensor 5 and the activity sensor 3, from a plurality of sensors of any type, and may be done during or after the treatment being provided, as explained with respect to the embodiment of the first aspect of the invention.

The treatment to be provided based on the received data associated to the muscle tone of the limb and/or to the at least one parameter associated with the user's activity can be computed according to several methods, such as algorithms, predefined look-up tables and or predefined thresholds as already mentioned in the embodiments of the first aspect of the invention.

Moreover, the algorithms, tables, and thresholds used for treatment determination may be preselected or customized according to individual user profiles as already mentioned in previous embodiments. By taking into account these personalized factors, the processor ensures that the determination of the treatment to be delivered by the action means 2 is not only accurate and effective but also safe and suitable for each individual user.

It is noted that, as shown in Fig. 5, there may be additional optional steps as will be described later.

Advantageously, a method according to the fourth aspect of the invention provides for a method for a personalized, non-invasive, cost-efficient and long-term solutions for movement disorders caused by central nervous system (CNS) injuries.

In a more preferred embodiment of the fourth aspect of the invention, and as shown in Fig. 5 the method further comprises the step 54 of determining the need to update the treatment to be provided based on the received data associated to the muscle tone of the limb and/or the at least one parameter associated with the user's activity.

The update on the treatment to be provided based on the received data associated to the muscle tone of the limb and/or to the at least one parameter associated with the user's activity can be computed according to several methods, such as algorithms, predefined look-up tables and or predefined thresholds as already mentioned in the embodiments of the first aspect of the invention.

All of the above are fully within the scope of the present disclosure and are considered to form the basis for alternative embodiments in which one or more combinations of the above-described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. A system configured for treating a stiffness or alleviating a movement disorder caused by a central nervous system injury in a limb of a user, the system comprising:
a. a device (10) comprising:
i. attachment means (1) configured for attaching the device (10) to the limb,
ii. an action means (2) configured to provide an external input to the limb,
iii. an activity sensor (3) configured to measure at least one parameter associated with the user's activity;
v. a controller (4) configured to control the action means (2) to provide the treatment in the form of external inputs to the limb; and
vi. at least one force sensor (5) configured to measure the muscle tone of the limb of the user,
b. and a processor (20) configured to:
i. receive data associated to at least one parameter associated with the user's activity from the activity sensor (3);
ii. receive data associated to the muscle tone of the limb of the user from the at least one force sensor (5); and
iii. determine the treatment to be provided by the action means (2) through the controller (4) based on the received data associated to the muscle tone of the limb and the received data associated to the at least one parameter associated with the user's activity.

2. The system according to claim 1, wherein the processor (20) is further configured to determine the need to update the treatment to be provided based on the received data associated to the muscle tone of the limb and to the at least one parameter associated with the user's activity.

3. The system according to claim 2, wherein the processor is further configured to determine the need to update the treatment to be provided during a treatment.

4. The system according to claims 2 or 3, wherein the processor is further configured to determine the need to update the treatment after a treatment has been performed.

5. The system according to any one of the previous claims, wherein the at least one force sensor (5) is a load sensor.

6. The system according to any one of the previous claims, wherein the at least one force sensor (5) is a current sensor configured to measure the current employed by the action means (2).

7. The system according to any one of the previous claims, wherein the system comprises at least two force sensors (5, 5'), the two force sensors being a load sensor and a current sensor configured to measure the current employed by the action means (2).

8. The system according to any one of the previous claims, wherein the processor (20) is comprised in an external device.

9. The system according to any one of the previous claims, wherein the processor (20) is comprised within the device (10).

10. The system according to any one of the previous claims, wherein the device (20) further comprises communication means (6) to indicate the treatment to be provided and/or the need to update the treatment to be provided.

11. The system according to any one of the previous claims, wherein the activity sensor (3) comprises an accelerometer.

12. The system according to any one of the previous claims, wherein the action means (2) is configured to provide pressure as an external input to the limb and wherein the processor (20) is configured to control the action means (2) to provide the treatment by pressing the limb at a pre-stablished pressure and at a pre-stablished depth.

13. The system according to claim 12, wherein the action means (2) comprises:
a. an actuator (22); and
b. a plunger (24);
and wherein the actuator (22) is configured to move the plunger (24) against a user's skin adjacent to the muscle of the limb.

14. Use of a system according to any one of claims 1 to 13 for treating or alleviating a movement disorder caused by a central nervous system injury in a limb of a user.

15. Computer program product connected to device according to any one of claims 1 to 13, comprising computer readable instructions which, when executed by a processor, cause the processor to execute the following steps:
a. receiving data associated to the muscle tone of the limb of the user from the force sensor (5) and data associated to at least one parameter associated with the user's activity from the activity sensor (3); and
b. determining the treatment to be provided based on the received data associated to the muscle tone of the limb and to the at least one parameter associated with the user's activity.
